# EUROPEAN PATENT APPLICATION

(11) **EP 4 102 358 A1**
(43) Date of publication of application: **14.12.2022**
(21) Application number: 20920976.6
(22) Date of filing: 17.11.2020
(51) Int. Cl.: G06F 9/44

(54) **APPLICATION PROGRAM GENERATING METHOD AND APPARATUS**

(30) Priority: 29.02.2020 CN 202010132098
(71) Applicant: HUAWEI TECHNOLOGIES CO., LTD., Shenzhen, Guangdong 518129 (CN)
(72) Inventor: CHEN, Yumei, Shenzhen, Guangdong 518129 (CN); CHEN, Yong, Shenzhen, Guangdong 518129 (CN); WU, Lian, Shenzhen, Guangdong 518129 (CN); LI, Junjin, Shenzhen, Guangdong 518129 (CN); TANG, Ye, Shenzhen, Guangdong 518129 (CN)
(74) Representative: Gill Jennings & Every LLP
(86) International application number: PCT/CN2020/129471
(87) International publication number: WO 2021/169426

(57) **Abstract**

An application program generation method and apparatus are provided. In the method, the application program generation apparatus determines one or more target modules, where the target module is configured to implement a different function in the application program, and the one or more target modules include a data management module, where the data management module is configured to invoke target data in an electronic device on which the application program is installed; reads, from a code library, software code corresponding to the target module; and generates the application program based on an application program basic framework and the software code corresponding to the target module, where the application program basic framework includes basic software code required for generating the application program. The method can automatically generate the application program, and after the application program is installed on the electronic device, the target data in the electronic device can be obtained, to facilitate collection of the target data.

## Description

### CROSS-REFERENCE TO RELATED APPLICATIONS

This application claims priority to Chinese Patent Application No. 202010132098.1, filed with the China National Intellectual Property Administration on February 29, 2020 and entitled "APPLICATION PROGRAM GENERATION METHOD AND APPARATUS", which is incorporated herein by reference in its entirety.

### TECHNICAL FIELD

This application relates to the field of computer technologies, and in particular to an application program generation method and apparatus.

### BACKGROUND

At present, sports and health research has become an increasingly concerned topic. A large quantity of health data needs to be collected from users as research samples for the sports and health research.

When conducting research, research institutions usually use professional medical devices to perform physical examinations on the users to obtain health data of the users. If many samples are needed, a large quantity of users need to be recruited to collect the data one by one, which is time-consuming. In addition, health data of the users cannot be monitored in real time.

Therefore, how to efficiently obtain health data of the users and carry out research is a problem on which the research institutions focus.

### SUMMARY

An objective of this application is to provide an application program generation method and apparatus for automatically generating an application program expected by a user.

The foregoing objective and other objectives are achieved by features in independent claims. Further implementations are embodied in the dependent claims, the specification, and the accompanying drawings.

According to a first aspect, an application program generation method is provided. The method is implemented by an application program generation platform, and the application program generation platform may be a server. The application program generation platform determines one or more target modules, where the target module is configured to implement a different function in an application program, the one or more target modules include a data management module, and the data management module is configured to invoke target data in an electronic device on which the application program is installed; read, from a code library, software code corresponding to the target module, and generate the application program based on an application program basic framework and the software code corresponding to the target module, where the application program basic framework includes basic software code required for generating the application program.

In other words, the application program generation platform may automatically generate the application program, to help a common user (a user who does not understand professional coding) generate the application program. In addition, the application program may be configured to collect the target data in the electronic device on which the application program is installed.

For example, the target data may be health data in the electronic device. If a research institute for health research generates the application program by using the application program generation method provided in embodiments of this application, and if the application program is installed on a plurality of electronic devices, the health data in the plurality of electronic devices can be obtained. This implements efficient collection of the health data, and facilitates health research performed by the research institute.

For example, the health data includes one or more of the following: sleep data, heart rate data, body fat data, blood pressure data, blood glucose data, step count data, or data collected by a motion sensor in the electronic device. It should be understood that the foregoing is an example of the health data, and is not a limitation. Other health data may also be included.

For example, the one or more target modules are preset, that is, the target module is determined in advance and does not need to be selected by the user. Alternatively, the target module may be selected by the user. To be specific, before the application program generation platform generates the application program, the user selects the target module, and the application program generation platform generates the application program based on the target module selected by the user. That is, the application program generation platform can generate the application program expected by the user, and the generated application program can implement a function corresponding to the module selected by the user.

For example, the target data is preset, that is, the target data is determined in advance and does not need to be selected by the user. Alternatively, the target data may be selected by the user. Assume that the target data is the health data, and the user selects the heart rate data or the like, the generated application program may obtain the heart rate data in the electronic device. That is, the application program generation platform can generate the application program expected by the user, and the generated application program can collect whatever data selected by the user.

In a possible design, after reading the software code corresponding to the target module from the code library, the application program generation platform may further receive customized content that is input by the user for the target module; and adjust, based on the customized content, the software code corresponding to the target module; and the generating the application program based on an application program basic framework and the software code corresponding to the target module includes: generating the application program based on the application program basic framework and adjusted software code corresponding to the target module.

That is, after selecting the target module, the user (for example, a research institute staff member) may further customize the target module, and add a unique design of the user, so that the generated application program can implement a function designed by the user.

In a possible design, before generating the application program based on the application program basic framework and the software code corresponding to the target module, the application program generation platform may further determine a component that corresponds to the target module in the application program basic framework, where the component is a directory in the application program basic framework; and store the software code corresponding to the target module in storage space corresponding to the component.

It may be understood that the application program basic framework includes the basic software code required for generating the application program, and the software code corresponding to the target module built on the basic framework (that is, storing the software code corresponding to the target module in the storage space corresponding to the component or directory corresponding to the target module on the basic framework). In this way, the application program is generated. In other words, the application program generation platform can automatically generate the application program, to help the common user (the user who does not understand professional coding) generate the application program. In addition, the application program may be configured to collect the target data in the electronic device on which the application program is installed. For example, the target data may be the health data in the electronic device. If the research institute for health research generates the application program by using the application program generation method provided in embodiments of this application, and if the application program is installed on the plurality of electronic devices, the health data in the plurality of electronic devices can be obtained. This implements efficient collection of the health data, and facilitates health research performed by the research institute.

In a possible design, the one or more target modules further include a permission confirmation module, and the permission confirmation module is configured to set a permission for the application program to access the target data in the electronic device.

It should be understood that, after the application program generated by the application program generation platform is installed on the electronic device, the user to which the electronic device belongs may authorize or not authorize the application program to access the data in the electronic device. It should be ensured that the target data in the electronic device is obtained with user authorization, which helps to improve user experience.

In a possible design, before the generating the application program based on the application program basic framework and the software code corresponding to the target module, the method further includes: providing a configuration interface, where the configuration interface includes a presentation interface of the target module; and adjusting the presentation interface based on an input operation of the user; and the generating the application program based on the application program basic framework and the software code corresponding to the target module includes: generating the application program based on the application program basic framework, the software code corresponding to the target module, and an adjusted presentation interface.

In other words, the user (for example, the research institute staff member) may adjust the presentation interface of the application program, which delivers high user experience.

According to a second aspect, an application generation apparatus is further provided, including: one or more processors; a memory; and one or more computer programs, where the one or more computer programs are stored in the memory, and the one or more computer programs include instructions, where when the instructions are executed by the apparatus, the apparatus is enabled to perform the following steps: determine one or more target modules, where the target module is configured to implement a different function in an application program, the one or more target modules include a data management module, and the data management module is configured to invoke target data in an electronic device on which the application program is installed; read, from a code library, software code corresponding to the target module; and generate the application program based on an application program basic framework and the software code corresponding to the target module, where the application program basic framework includes basic software code required for generating the application program.

In a possible design, when the instructions are executed by the apparatus, the apparatus is enabled to further perform the following steps: receive customized content that is input by a user for the target module; adjust, based on the customized content, the software code corresponding to the target module; and generate the application program based on the application program basic framework, and adjusted software code corresponding to the target module.

In a possible design, when the instructions are executed by the apparatus, the apparatus is enabled to further perform the following steps: determine a component corresponding to the target module in the application program basic framework, where the component is a directory in the application program basic framework; and store the software code corresponding to the target module in storage space corresponding to the component.

In a possible design, the one or more target modules further include a permission confirmation module, and the permission confirmation module is configured to set a permission for the application program to access the target data in the electronic device.

In a possible design, when the instructions are executed by the apparatus, the apparatus is enabled to further perform the following steps: display a configuration interface, where the configuration interface includes a presentation interface of the target module; adjust the presentation interface based on an input operation of the user; and generate the application program based on the application program basic framework, the software code corresponding to the target module, and an adjusted presentation interface.

In a possible design, the one or more target modules are preset or selected by the user; and the target data is preset or determined by the user.

In a possible design, the target data includes health data.

In a possible design, the health data includes one or more of the following:
sleep data, heart rate data, body fat data, blood pressure data, blood glucose data, step count data, or data collected by a motion sensor in the electronic device.

According to a third aspect, an application generation apparatus is further provided, including: a module/unit for performing the method in the first aspect or any possible design of the first aspect. The module/unit may be implemented by hardware, or may be implemented by corresponding software implemented by the hardware.

According to a fourth aspect, a communications system is further provided, including: an application program generation apparatus, configured to implement the method in the first aspect; and an electronic device, configured to install an application program generated by the application program generation apparatus. The application generation apparatus may be a server.

According to a fifth aspect, a chip is further provided. The chip is coupled to a memory in a computer, so that when running, the chip invokes program instructions stored in the memory to implement the method according to the first aspect.

According to a sixth aspect, a computer-readable storage medium is further provided, and the computer-readable storage medium includes a computer program. When the computer program is run on a computer, the computer is enabled to perform the method according to the first aspect.

According to a seventh aspect, a computer program product is further provided. The computer program product includes instructions. When the instructions are run on a computer, the computer is enabled to perform the method according to the first aspect.

For beneficial effects of the second aspect to the seventh aspect, refer to the beneficial effects of the first aspect. Details are not described again.

### BRIEF DESCRIPTION OF DRAWINGS

FIG. 1 is a functional block diagram of an application program generation platform according to an embodiment of this application;
FIG. 2 is a schematic diagram of an application scenario according to an embodiment of this application;
FIG. 3 is a schematic flowchart of an application program generation method according to an embodiment of this application;
FIG. 4 is a schematic diagram of a display interface provided by an application program generation platform according to an embodiment of this application;
FIG. 5 is a schematic diagram of an application program basic framework according to an embodiment of this application;
FIG. 6 is a schematic diagram of a combination of an application program basic framework and a target module according to an embodiment of this application;
FIG. 7 is a schematic diagram of a configuration interface provided by an application program generation platform according to an embodiment of this application; and
FIG. 8 is a schematic diagram of a structure of an apparatus according to an embodiment of this application.

### DESCRIPTION OF EMBODIMENTS

A technical solution in embodiments of this application is described in detail below with reference to the accompanying drawings of the following embodiments of this application.

The following describes some terms in embodiments of this application, to facilitate understanding of a person skilled in the art.
(1). An application program, or an application (application, app for short), in embodiments of this application is a software program that can implement one or more specific functions. For example, the application may be a camera application, an SMS message application, a mailbox application, WeChat (WeChat), WhatsApp Messenger, Line (Line), Instagram (Instagram), Kakao Talk, DingTalk, or the like. An application program generation method provided in embodiments of this application can be used to automatically generate an application program.

An application program interface (application program interface, API) may be understood as an invocation interface left by an operating system of an electronic device for an application program, and the application program enables, by invoking the API interface, the operating system to execute a command of the application program. Generally, the operating system of the electronic device may provide a framework (framework). The framework includes a plurality of API interfaces, each API interface is configured to implement a specific function, and different application programs may separately invoke corresponding API interfaces in the framework, to implement the specific functions via the API interfaces.

(2). An electronic device in embodiments of this application may be, for example, a mobile phone (mobile phone), a tablet, a laptop, a palmtop, a mobile internet device (mobile internet device, MID), and the like. The electronic device may also be a wearable device, like a watch, a band, glasses, a headset, a smart helmet, a necklace, and so on. The application program generated by the application program generation method provided in embodiments of this application may be installed in any one of the foregoing electronic devices.

(3). The application program generation platform in embodiments of this application is a platform configured to generate an application program. The platform may be a server, or the platform may be understood as a client, for example, an application, configured to generate the application program; or an online platform, for example, a web page. Take a web page as an example. When a user wants to generate an application program, the user inputs a web page link in a device (for example, a laptop), to enter the web page. The web page includes identifiers of different modules, such as a data management module and a questionnaire survey module, and the different modules are configured to implement different specific functions. Take the data management module as an example. The data management module may be configured to obtain target data in the electronic device on which an application program is installed. That is, assume that the user selects the data management module to generate the application program, after the application program is installed on the electronic device, the application program may obtain the target data in the electronic device. The target data is, for example, health data in the electronic device, or other data in the electronic device. This is not limited in embodiments of this application. The following uses health data as an example.

Software code corresponding to different modules may be written by a professional programmer, and pre-stored in the platform. Therefore, the user may select a module of interest from the web page, and the application program generation platform generates an application program based on the software code corresponding to the module selected by the user, so that the application program can implement the specific function corresponding to the module selected by the user. Therefore, an application program can also be generated by the user who does not have a professional coding capability, and it is convenient for the user to operate. Still take the data management module as an example. Assume that the software code of the data management module compiled by the professional programmer in advance is used to obtain the health data in the electronic device. If the user selects the data management module to generate an application program, the application program may obtain the health data in the electronic device on which the application program is installed. Similarly, assume that the software code of the data management module compiled by the professional programmer in advance is used to obtain other data in the electronic device. If the user selects the data management module to generate an application program, the application program obtains other data in the electronic device on which the application program is installed.

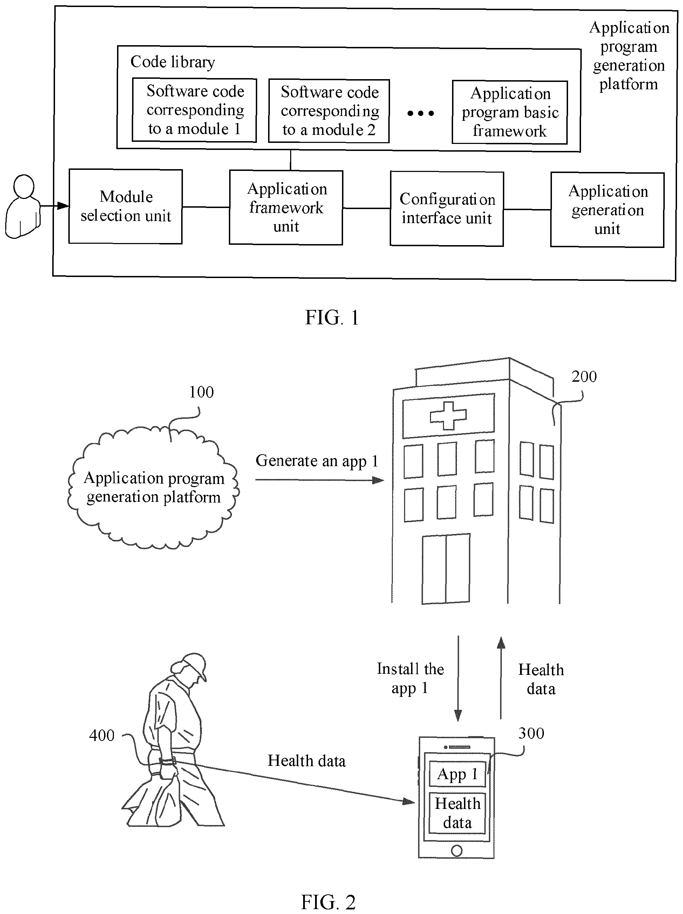

FIG. 1 is a functional block diagram of an example of an application program generation platform according to an embodiment of this application. As shown in FIG. 1, the application program generation platform includes a module selection unit, an application framework unit, a configuration interface unit, an application generation unit, and a code library. The module selection unit is configured to determine a target module from a plurality of modules. Take the foregoing web page as an example. After inputting a web page link in a device to enter the web page, the user selects an identifier of the target module from a plurality of module identifiers provided on the web page. The device sends the identifier of the target module to the application program generation platform, and an application generation module determines the target module based on the identifier.

The application framework module adjusts an application program basic framework based on the determined target module. The code library stores software code corresponding to each module. The modules are, for example, a data management module and a questionnaire survey module, and each module is configured to implement a specific function. The code library may further include the application program basic framework. The basic framework may be understood as basic software code used to generate an application program. When an application program needs to be generated, the software code corresponding to the target module may be built on the application program basic framework. The configuration interface unit is configured to provide a configuration interface, where the configuration interface includes a presentation interface of each target module, so that it is convenient for the user to adjust the interface. The application generation module is configured to generate the application program based on an adjusted basic framework and an adjusted presentation interface.

(4). In embodiments of this application, "at least one" means one or more, and "a plurality of' means two or more. In addition, it should be understood that in description of this application, terms such as "first" and "second" are merely used for differentiation and description, but should not be understood as an indication or implication of relative importance, or an indication or implication of a sequence.

The following describes a possible application scenario of an application program generation method provided in embodiments of this application. In this application scenario, for example, a user (for example, the foregoing health research institute) wants to generate an application program that can obtain target data, that is, health data, from an electronic device.

As described above, when conducting health research, the research institute needs to collect the health data from a large quantity of users. An existing practice is that the research institute collects the health data artificially, that is, using a professional medical device to perform physical examinations on the users one by one to collect the health data. This is a tedious process. The research institute may generate, by using the method provided in embodiments of this application, the application program configured to obtain the health data in the electronic device.

FIG. 2 is a schematic diagram of an application scenario according to an embodiment of this application. As shown in FIG. 2, the application scenario includes an application program generation platform 100, a research institute 200, an electronic device 300, and a wearable device 400. The application program generation platform 100 is configured to generate an application program. The research institute 200 is, for example, a medical research institute (for example, a hospital), or the like. The electronic device 300 may be a device such as a mobile phone (mobile phone), a tablet, or a laptop. The wearable device 400 is, for example, a watch, a band, a smart helmet, a headset, glasses, a necklace, or the like.

The application program generation platform 100 may provide a web page link. A research institute staff member enters, via the device (for example, the laptop of the staff member), a web page corresponding to the web page link, where the web page provides different module identifiers, and the staff member selects one or more target modules from the web page, which may include a data management module configured to obtain health data. The web page further includes a key for an indication to generate an application program automatically. When detecting an operation on the key, the device sends, to the application program generation platform 100, instructions to generate an application program, and the application program generation platform 100 generates an app 1 based on the target module selected by the staff member.

The application program may be launched to an application market, or may be provided to a user in another manner (for example, via a QR code). After the user downloads and installs the app 1 by using the electronic device 300, the app 1 may obtain the health data in the electronic device 300, and then send the health data to a background of the research institute 200, to facilitate research performed by the research institute. The health data in the electronic device 300 may be obtained from the wearable device 400 worn by the user, that is, a sensor module is installed in the wearable device 400, and the sensor module may collect the health data of a human body, for example, heart rate data. The wearable device 400 sends the collected health data to the electronic device 300. In some other embodiments, the wearable device 400 may also download the app 1, and in this case, the app 1 obtains the health data in the wearable device 400, and reports the health data to the background of the research institute. The health data mentioned herein may include but is not limited to sleep data, heart rate data, step count data, body fat data, blood pressure data, blood glucose data, and the like of the user.

Currently, the wearable device that can collect the health data of the user is gradually popularized. Therefore, the research institute can obtain the health data in the wearable device from a large quantity of users (with user authorization) by using the application program, to carry out a more efficient research into a research result.

In addition, the generated application program may provide a health service for the user, for example, a beneficial service like popularization of science articles and disease warning. Based on these services, if more users choose to download the application program to enjoy the services brought by the application program, the research institute can expand a sample size by collecting the health data from more users.

It should be noted that an application program generation method provided in embodiments of this application is further applicable to other scenarios. The application program generation platform has a plurality of modules, and the user may select any module from the plurality of modules to form the target module to generate the application program. Details are not listed one by one in embodiments of this application. The following uses the application scenario shown in FIG. 2 as an example for description.

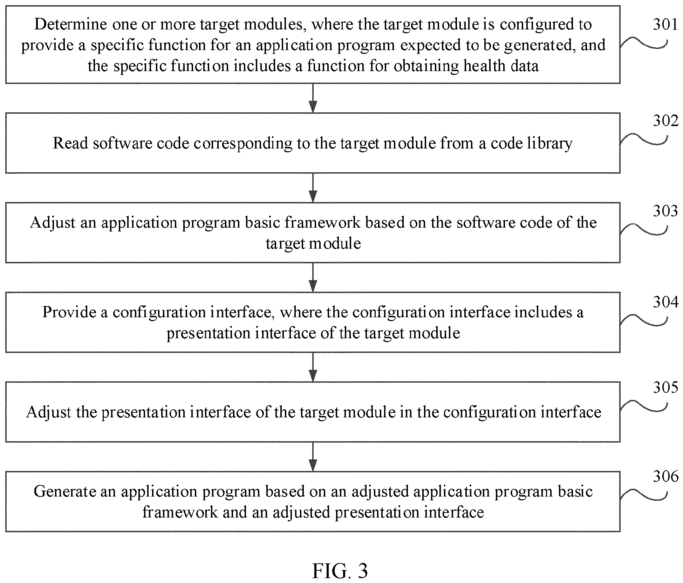

FIG. 3 is a schematic flowchart of an example of an application program generation method according to an embodiment of this application. The method may be implemented by an application program generation platform. As shown in FIG. 3, a procedure of the method includes the following steps.

Step 301: The application program generation platform determines one or more target modules, where different modules are configured to implement different specific functions of an application program. The one or more target modules include, for example, a data management module, a privacy management module, an account management module, or the like. The data management module can obtain health data in an electronic device on which the application program is installed.

In some embodiments, the target module may be selected by a user. Take the application scenario shown in FIG. 2 as an example, the user herein may be understood as a research institute staff member in the application scenario shown in FIG. 2.

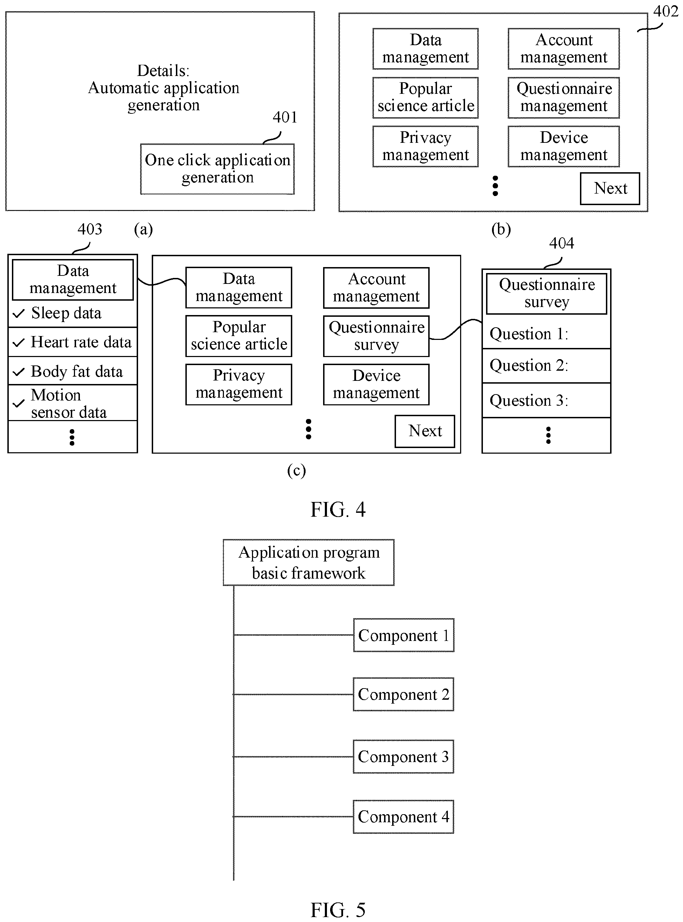

For example, the research institute staff member uses a terminal (a terminal used by the staff member) to enter a web page provided by the application program generation platform. For example, (a) in FIG. 4 shows a schematic diagram of an example of the web page provided by the application program generation platform. The web page includes a key 401 "one click application generation". When the electronic device of the user detects the key, a module selection interface 402 is displayed. The module selection interface 402 includes identifiers of a plurality of modules, and the identifiers may be used to help the user (the research institute staff member) distinguish different modules, to select the target module from the modules. Six module identifiers "data management", "account management", "popular science article", "questionnaire survey", "privacy management", and "device management" are shown in the figure as an example. In actual application, more or fewer module identifiers than those in the figure may be included.

In some embodiments, modules may be customized. Customization herein may be understood as that the research institute staff member may design, based on a requirement of the staff member, a function that can be implemented by each module. The following uses examples to describe a module customization process.

Take the questionnaire survey module as an example, the questionnaire survey module is configured to implement a questionnaire survey function. The process of customizing the questionnaire survey module may be understood as customizing a question corresponding to the module. For example, assume that the user selects the questionnaire survey module (for example, the user clicks an identifier of the questionnaire survey module) in (b) in FIG. 4, an interface 404 in (c) in FIG. 4 may be displayed. The interface 404 includes a plurality of question templates, and the user may add, delete, or design specific content of each question template based on the requirement. The application program generation platform generates the application program based on these questions. After the application program is downloaded and installed on the electronic device, the application program displays the questions, to collect a survey result of the user on different questions.

Take the data management module as an example. The data management module is configured to implement data management, where the data management includes, for example, obtaining and managing target data. The target data is, for example, health data. In some embodiments, the health data is selected by the user (the research institute staff member). For example, assume that the user selects the data management module (for example, the user clicks an identifier of the data management module) in (b) in FIG. 4, an interface 403 in (c) in FIG. 4 may be displayed. The interface 403 includes a plurality of data options, for example, options such as sleep data, heart rate data, body fat data, motion sensor data, and the like. The user can select the target data from the plurality of data options. After the user selects the target data, the generated application program can obtain the target data. Assume that the user selects the sleep data. After the application program is generated, if the electronic device downloads and installs the application program, the application program can obtain the sleep data in the electronic device. In some other embodiments, the health data is preset. For example, after the user (the research institute staff member) selects the data management module, the data management module selects the sleep data, the heart rate data, the body fat data, and the motion sensor data by default, and no user selection is required.

The account management module is configured to manage an account, the popular science article module is configured to provide a popular science article, and the privacy management module is configured to manage user privacy. Each module can be customized, and is not described one by one herein.

After selecting and customizing the target module, the research institute staff member may click a "next" key in (c) in FIG. 4, and the application program generation platform performs step 302.

In some other embodiments, the target module may alternatively be preset. Take (a) in FIG. 4 as an example. When the user clicks the key 401 "one click application generation", the application program generation platform automatically selects the preset target module from a plurality of modules, and no user selection is required. For example, a commonly used module is automatically selected as the target module, and the commonly used module is, for example, a module whose quantity of selection times is greater than a preset quantity of times; or all modules are automatically selected as the target modules, and the like. This is not limited in embodiments of this application.

Step 302: The application program generation platform reads software code corresponding to the target module from a code library. The software code corresponding to each module herein may be understood as the software code on which an implementation of the function corresponding to each module depends. The software code may be compiled by a programmer in advance, and stored in the code library.

It may be understood that a mapping relationship between a module identifier and a storage address of the software code exists on the application program generation platform. In step 301, after determining the target module, the application program generation platform determines the storage address of the corresponding software code based on the identifier of the target module, and then reads, based on the storage address, the software code corresponding to the target module.

Optionally, after reading the software code corresponding to the target module from the code library, the application program generation platform may further properly adjust the software code of the target module. It may be understood that the software code corresponding to the target module and stored in the code library may be understood as basic code for implementing the target module. If the application program generation platform confirms that the user customizes a target module, content customized by the user may be added to the basic code corresponding to the target module. Take the questionnaire survey module as an example. After the user customizes a question in the questionnaire survey module, the application program generation platform may add content of the customized question to basic code corresponding to the questionnaire survey module.

Step 303. Adjust an application program basic framework based on the software code of the target module. The application program basic framework herein may be understood as basic software code required for generating an application program. The application program generation platform pre-stores the application program basic framework, and when an application program needs to be generated, the application generation program builds the application program on the basic framework. For example, the target module selected by the user is built on the application program basic framework.

For ease of understanding, FIG. 5 is a schematic diagram of an application program basic framework. As shown in FIG. 5, the application program basic framework includes a plurality of components. The component may be understood as a directory in the application program basic framework. Different components may correspond to different modules. Alternatively, the component may be understood as a bridge between the application program basic framework and a corresponding target module. The application program basic framework invokes software code corresponding to the corresponding target module by using the component, to provide a function of the corresponding target module for a user.

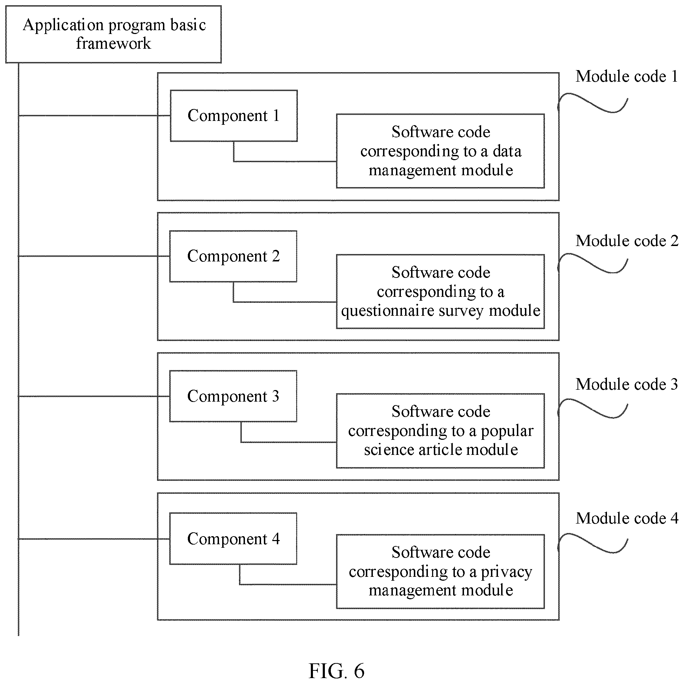

Take FIG. 5 as an example, a component 1 corresponds to a data management module, a component 2 corresponds to a questionnaire survey module, a component 3 corresponds to a popular science article module, and a component 4 corresponds to a privacy management module. It may be understood that, in actual application, the application program basic framework may include more or fewer components than those in FIG. 5. A process in which an application program generation platform builds the target module on the application program basic framework may be combining the software code of the target module and the component corresponding to the target module into module code. Refer to FIG. 6. Software code of the data management module and the corresponding component 1 are combined into module code 1. Software code of the questionnaire survey module and the corresponding component 2 are combined into module code 2.

Combining the target module and the corresponding component into the module code may be understood as storing the software code corresponding to the target module into storage space of the corresponding component (or referred to as the directory).

Still take FIG. 6 as an example. The software code of the data management module may be stored in storage space corresponding to a component, for example, the component 1, corresponding to the data management module, to obtain the module code 1. The software code of the questionnaire survey module may be stored in storage space corresponding to a component, for example, the component 2, corresponding to the questionnaire survey module, to obtain the module code 2.

The foregoing different module code includes invocation modules configured to invoke different APIs, and the different APIs are configured to invoke different target data.

Take the module code 1 combined by the data management module and the component 1 as an example. The module code 1 includes an invocation module configured to invoke a first API. The first API is configured to invoke target data in an electronic device (an electronic device on which a generated application program is to be installed), for example, the target data selected by the user in FIG. 4. For example, the target data may be sleep data, heart rate data, body fat data, motion sensor data, and the like.

In some embodiments, the one or more target modules determined by the application program generation platform in step 301 may further include a permission confirmation module. The permission confirmation module is configured to set a permission to access, for example, health data, a camera application, contacts, a picture, and the like. When the permission confirmation module confirms user authorization to access the health data, the invocation module in the foregoing module code 1 is invoked, to invoke the first API to obtain the health data in the electronic device.

Take the module code 2 combined by the questionnaire survey module and the component 2 as an example. The module code 2 includes an invocation module configured to invoke a second API, where the second API is configured to invoke questionnaire survey content on a display, for example, the content of the question customized by the user in FIG. 4, in the electronic device (the electronic device on which the generated application program is to be installed).

It may be understood that a quantity of components included in the application program basic framework may be greater than a quantity of target modules selected by the user. Assume that the application program basic framework includes 10 components by default, corresponding to 10 modules. If there are only six target modules determined in step 301, the six components in the application program basic framework may be used, and the other four components may not be used. For example, the four components may be deleted. This is not limited in embodiments of this application.

Step 304: Provide a configuration interface, where the configuration interface includes a presentation interface of the target module. The configuration interface may facilitate customization of a presentation interface of each target module by the user.

Step 305: Adjust the presentation interface of the target module in the configuration interface.

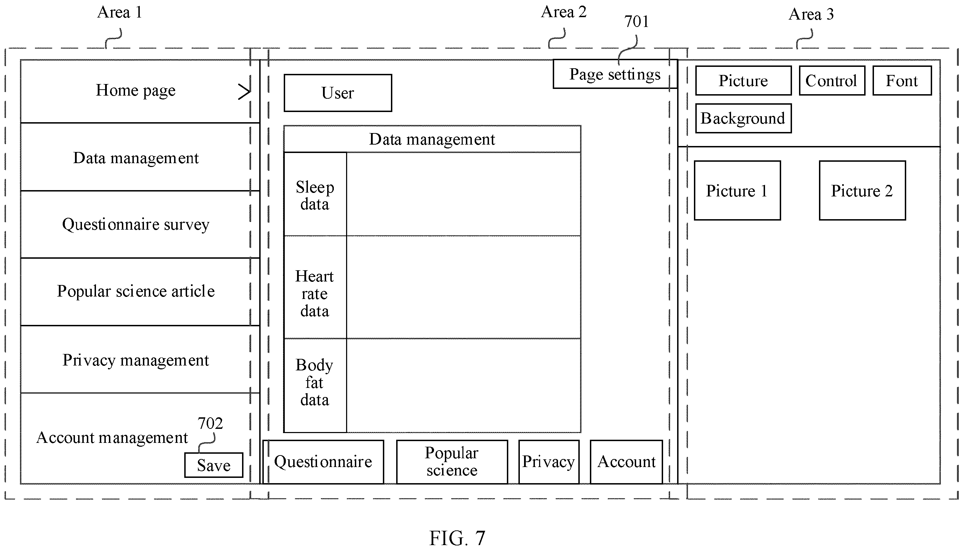

For example, FIG. 7 is a schematic diagram of an example of a configuration interface. As shown in FIG. 7, an area 1 includes identifiers of each target module and a home page. When a user selects the identifier of the home page, a presentation interface of the home page is displayed in an area 2, and the user may adjust the presentation page of the home page. For example, the user can adjust a font size, background, picture, layout of pictures, and the like on the home page. For example, the presentation interface of the home page in the area 2 includes a "page settings" key 701. When the user clicks the key 701, a selection interface of page elements is displayed in an area 3, and the user may select, in the interface in the area 3, the picture, background, or the like to decorate the presentation interface of the home page.

For another example, when the user selects an identifier of a data management interface in the area 1, a presentation interface of the data management interface is displayed in the area 2, and the user may adjust the presentation interface. In other words, the user may adjust a presentation interface of each target module based on a preference of the user. After adjusting the presentation interface, the user may click a "save" key 702. The application program generation platform performs step 306.

Step 306: Generate an application program based on an adjusted application program basic framework and an adjusted presentation interface.

In some embodiments, before step 306, the user (for example, a research institute staff member) may further choose to generate application programs applicable to different operating systems, for example, Android or iOS. Assume that the user selects the Android operating system, a generated application program is applicable for download and installation by an electronic device with the Android operating system.

After generating the application program, the application program generation platform may launch an installation package of the application program to an application market for the user to download. Certainly, the research institute may also provide the installation package of the generated application program for the user in other manners, for example, provide a QR code, a website link, and the like for installing the application program. It should be understood that the QR code, website link, or the like may be set on an advertising platform that is convenient for the user to view. Details are not described in embodiments of this application.

Optionally, in the foregoing process of generating the application program by the application program generation platform, the research institute staff member selects target data, for example, sleep data, heart rate data, body fat data, and motion sensor data in FIG. 4. Therefore, the generated application program may obtain the target data in the electronic device on which the application program is installed. It should be noted that the sleep data, the heart rate data, the step count data, or the like is usually obtained based on analysis of data collected by a motion sensor. In other words, the data collected by the motion sensor may be understood as raw data (or referred to as initial data), and the sleep data, the heart rate data, the step count data, or the like is obtained after the raw data is analyzed. That is, the generated application program may also obtain the raw data in the electronic device, and/or the sleep data, the heart rate data, the step count data, or the like that is obtained by using the raw data, so that the research institute can conduct research.

In some embodiments, after the generated application program is installed on the electronic device of the user, the user may authorize the application program to, for example, access data. In other words, the application program may obtain the data that is in the electronic device and that is accessed with the user authorization. For example, if the user authorizes the application program to access the heart rate data, but does not authorize the application program to access the sleep data, the application program can obtain the heart rate data, but cannot obtain the sleep data. Specifically, when running the application program, the electronic device of the user runs the module code 1 corresponding to the data management module in the application program, where the invocation module in the module code 1 invokes the first API, and the first API is configured to invoke the data that is in the electronic device and that is accessed with the user authorization, for example, the heart rate data.

In some other embodiments, a background of the research institute may send a popular science article and the like to the application program, to popularize physical health knowledge information to the user.

In some other embodiments, the background of the research institute may further provide potential disease risk information based on the health data of the user, and provide a corresponding solution, to help the user prevent a disease as much as possible.

The implementations of this application may be combined randomly to achieve different technical effects.

In the foregoing embodiments provided by this application, the method provided in embodiments of this application is described from a perspective of the application program generation platform (for example, a server) as an execution body. To implement functions in the method provided in embodiments of this application, the electronic device may include a hardware structure and/or a software module, and implement the functions in a form of the hardware structure, the software module, or a combination of the hardware structure and the software module. Whether a specific function in the foregoing functions is performed by the hardware structure, the software module, or the combination of the hardware structure and the software module depends on specific applications and design constraints of the technical solution.

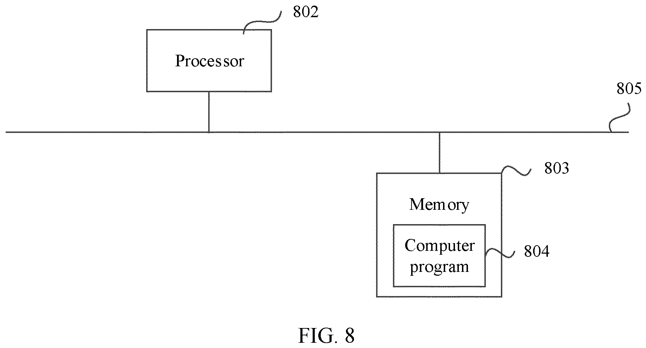

Refer to FIG. 8. Some other embodiments of this application disclose a schematic diagram of an apparatus. The apparatus may be the foregoing application program generation platform, for example, a server. The apparatus may include one or more processors 802, and one or more memories 803. The foregoing devices may be connected by one or more communications buses 805. The one or more computer programs 804 are stored in the memories 803 and are configured to be executed by the one or more processors 802. The one or more computer programs 804 include instructions, and the instructions may be used to perform the steps in FIG. 3 to FIG. 7 and the corresponding embodiments.

It should be noted that, in embodiments of this application, unit division is an example, and is merely logical function division. In actual implementation, other division may be used. Functional units in embodiments of the present invention may be integrated into one processing unit, or each of the units may exist alone physically, or two or more units are integrated into one unit. For example, in the foregoing embodiments, a first obtaining unit and a second obtaining unit may be a same unit, or may be different units. The integrated unit may be implemented in a form of hardware, or may be implemented in a form of a software functional unit.

Terms used in the foregoing embodiments are merely for a purpose of describing specific embodiments, but are not intended to limit this application. The terms "one", "a" and "this" of singular forms used in this specification and the appended claims of this application are also intended to include expressions such as "one or more", unless otherwise specified in the context clearly. It should be further understood that, in embodiments of this application, "one or more" refers to one, two, or more, and the term "and/or" describes an association between associated objects, and indicates that three relationships may exist. For example, A and/or B may indicate the following cases: Only A exists, both A and B exist, and only B exists, where A and B may be singular or plural. The character "/" generally indicates an "or" relationship between the associated objects.

Reference to "an embodiment", "some embodiments", or the like described in this specification indicates that one or more embodiments of this application include a specific feature, structure, or characteristic described with reference to embodiments. Therefore, statements such as "in an embodiment", "in some embodiments", "in some other embodiments", and "in other embodiments" that appear at different places in this specification do not necessarily mean referring to a same embodiment, instead, they mean "one or more but not all of the embodiments", unless otherwise specifically emphasized. The terms "include", "contain", "have", and their variants all mean "include but are not limited to", unless otherwise specifically emphasized.

According to context, the term "when..." or "after..." used in the foregoing embodiments may be interpreted as a meaning of "if...", "after...", "in response to determining...", or "in response to detecting...". Similarly, according to the context, the phrase "when it is determined that..." or "if (a stated condition or event) is detected" may be interpreted as a meaning of "if it is determined that...", "in response to determining... ", "when (a stated condition or event) is detected", or "in response to detecting (a stated condition or event)". In addition, in the foregoing embodiments, relationship terms such as first and second are used to distinguish one entity from another entity, but do not limit any actual relationship and sequence between these entities.

All or some of the foregoing embodiments may be implemented by using software, hardware, firmware, or any combination thereof. When software is used to implement the embodiments, all or a part of the embodiments may be implemented in a form of a computer program product. The computer program product includes one or more computer instructions. When the computer program instructions are loaded and executed on a computer, all or some of procedures or functions according to embodiments of the present invention are generated. The computer may be a general-purpose computer, a dedicated computer, a computer network, or another programmable apparatus. The computer instructions may be stored in a computer-readable storage medium, or may be transmitted from one computer-readable storage medium to another computer-readable storage medium. For example, the computer instructions may be transmitted from one website, computer, server, or data center to another website, computer, server, or data center in a wired (for example, a coaxial cable, an optical fiber, or a digital subscriber line (DSL)) or wireless (for example, infrared, radio, or microwave) manner. The computer-readable storage medium may be any usable medium accessible by a computer, or a data storage device, such as a server or a data center, integrating one or more usable media. The available medium may be a magnetic medium (for example, a floppy disk, a hard disk, or a magnetic tape), an optical medium (for example, a DVD), a semiconductor medium (for example, a solid state disk Solid State Disk (SSD)), or the like.

It should be noted that a part of the present patent application document includes content protected by the copyright. The copyright owner reserves the copyright except copies made for the patent documents or the recorded content of the patent documents in the Patent Office.

## Claims

1. An application program generation method, comprising:
determining one or more target modules, wherein the target module is configured to implement a different function in an application program, the one or more target modules comprise a data management module, and the data management module is configured to invoke target data in an electronic device on which the application program is installed;
reading, from a code library, software code corresponding to the target module; and
generating the application program based on an application program basic framework and the software code corresponding to the target module, wherein the application program basic framework comprises basic software code required for generating the application program.

2. The method according to claim 1, wherein after the reading, from a code library, software code corresponding to the target module, the method further comprises:
receiving customized content input by a user for the target module;
adjusting, based on the customized content, the software code corresponding to the target module; and
the generating the application program based on an application program basic framework and the software code corresponding to the target module comprises:
generating the application program based on the application program basic framework and adjusted software code corresponding to the target module.

3. The method according to claim 1 or 2, wherein before the generating the application program based on the application program basic framework and the software code corresponding to the target module, the method further comprises:
determining a component corresponding to the target module in the application program basic framework, wherein the component is a directory in the application program basic framework; and
storing the software code corresponding to the target module in storage space corresponding to the component.

4. The method according to any one of claims 1 to 3, wherein the one or more target modules further comprise a permission confirmation module, and the permission confirmation module is configured to set a permission for the application program to access the target data in the electronic device.

5. The method according to any one of claims 1 to 4, wherein before the generating the application program based on the application program basic framework and the software code corresponding to the target module, the method further comprises:
displaying a configuration interface, wherein the configuration interface comprises a presentation interface of the target module;
adjusting the presentation interface based on an input operation of the user; and
the generating the application program based on an application program basic framework and the software code corresponding to the target module comprises:
generating the application program based on the application program basic framework, the software code corresponding to the target module, and an adjusted presentation interface.

6. The method according to any one of claims 1 to 5, wherein the one or more target modules are preset or selected by the user; and the target data is preset or determined by the user.

7. The method according to any one of claims 1 to 6, wherein the target data comprises health data.

8. The method according to claim 7, wherein the health data comprises one or more of the following:
sleep data, heart rate data, body fat data, blood pressure data, blood glucose data, step count data, or data collected by a motion sensor in the electronic device.

9. An application program generation apparatus, comprising:
one or more processors;
a memory; and
one or more computer programs, wherein the one or more computer programs are stored in the memory, the one or more computer programs comprise instructions, and when the instructions are executed by the apparatus, the apparatus is enabled to perform the following steps:
determine one or more target modules, wherein the target module is configured to implement a different function in an application program, the one or more target modules comprise a data management module, and the data management module is configured to invoke target data in an electronic device on which the application program is installed;
read, from a code library, software code corresponding to the target module; and
generate the application program based on an application program basic framework and the software code corresponding to the target module, wherein the application program basic framework comprises basic software code required for generating the application program.

10. The apparatus according to claim 9, wherein when the instructions are executed by the apparatus, the apparatus is enabled to further perform the following steps:
receive customized content that is input by a user for the target module;
adjust, based on the customized content, the software code corresponding to the target module; and
generate the application program based on the application program basic framework and adjusted software code corresponding to the target module.

11. The apparatus according to claim 9 or 10, wherein when the instructions are executed by the apparatus, the apparatus is enabled to further perform the following steps:
determine a component corresponding to the target module in the application program basic framework, wherein the component is a directory in the application program basic framework; and
store the software code corresponding to the target module in storage space corresponding to the component.

12. The apparatus according to any one of claims 9 to 11, wherein the one or more target modules further comprise a permission confirmation module, and the permission confirmation module is configured to set a permission for the application program to access the target data in the electronic device.

13. The apparatus according to any one of claims 9 to 12, wherein when the instructions are executed by the apparatus, the apparatus is enabled to further perform the following steps:
display a configuration interface, wherein the configuration interface comprises a presentation interface of the target module;
adjust the presentation interface based on an input operation of the user, and
generate the application program based on the application program basic framework, the software code corresponding to the target module, and an adjusted presentation interface.

14. The apparatus according to any one of claims 9 to 13, wherein the one or more target modules are preset or selected by the user; and the target data is preset or determined by the user.

15. The apparatus according to any one of claims 9 to 14, wherein the target data comprises health data.

16. The apparatus according to claim 15, wherein the health data comprises one or more of the following:
sleep data, heart rate data, body fat data, blood pressure data, blood glucose data, step count data, or data collected by a motion sensor in the electronic device.

17. A communications system, comprising:
the application program generation apparatus according to any one of claims 9 to 16; and
an electronic device, wherein the electronic device is configured to install and run an application program generated by the application program generation apparatus.

18. A computer-readable storage medium, wherein the computer-readable storage medium comprises a computer program; and when the computer program is run on a computer, the computer is enabled to perform the method according to any one of claims 1 to 8.
